# EUROPEAN PATENT APPLICATION

(11) **EP 4 603 146 A2**
(43) Date of publication of application: **20.08.2025**
(21) Application number: 25187066.3
(22) Date of filing: 10.09.2020
(51) Int. Cl.: A61P 31/04

(54) **COMBINATION OF ZIDOVUDINE AND FLUOROQUINOLONE ANTIBIOTIC**

(30) Priority: 10.09.2019 GB 201913068
(62) Divisional of application: 20772392.5
(71) Applicant: Helperby Therapeutics Limited, London, Greater London WC2A 3LH (GB)
(72) Inventor: COATES, Anthony, London, SW17 0RE (GB); HU, Yanmin, London, SW17 0RE (GB)
(74) Representative: HGF

(57) **Abstract**

The present invention relates to a combination comprising zidovudine or a pharmaceutically acceptable derivative thereof and a fluoroquinolone antibiotic or a pharmaceutically acceptable derivative or prodrug thereof, for use in treating a microbial infection, particularly a bacterial infection such as a urinary tract infection.

## Description

### Field of the Invention

The present invention relates to the combination of zidovudine or a pharmaceutically acceptable derivative thereof with a fluoroquinolone antibiotic compound or a pharmaceutically acceptable derivative or prodrug thereof selected from the group defined herein, for use in the treatment of microbial infections. In particular, it relates to the use of such combinations to kill multiplying (i.e. log phase) microorganisms associated with microbial infections, e.g. Gram-negative bacterial infections including urinary tract infections.

### Background

Before the introduction of antibiotics, patients suffering from acute microbial infections (e.g. tuberculosis or pneumonia) had a low chance of survival. For example, mortality from tuberculosis was around 50%. Although the introduction of antimicrobial agents in the 1940s and 1950s rapidly changed this picture, bacteria have responded by progressively gaining resistance to commonly used antibiotics. Now, every country in the world has antibioticresistant bacteria.

Indeed, more than 70% of bacteria that give rise to hospital acquired infections in the USA resist at least one of the main antimicrobial agents that are typically used to fight infection (Nature Reviews, Drug Discovery, 1, 895-910 (2002)). The World Health Organization has therefore classified antimicrobial resistance as a "serious threat [that] is no longer a prediction for the future, it is happening right now in every region of the world and has the potential to affect anyone, of any age, in any country" ("Antimicrobial resistance: global report on surveillance", The World Health Organization, April 2014).

One group of antibiotics which is facing critical resistance problems is the compounds used to treat urinary tract infections (UTls) and specifically genitourinary infections. In a recent report from Public Health England, it was noted that antimicrobial resistance was common in more than 1 million urinary tract infections caused by bacteria identified in NHS laboratories in 2016 (« English Surveillance Programme for Antimicrobial Utilisation and Resistance (ESPAUR) » (2017)).

A solution to the growing problem of resistant bacteria causing urinary tract infections is therefore desperately needed.

In a lot of cases, UTIs are treated with a short course of antibiotics taken by mouth. The fluoroquinolones are often used for genitourinary infections and are widely used in the treatment of hospital-acquired infections associated with urinary catheters. One example of a fluoroquinolone is ciprofloxacin which is one of the most widely used antibiotics worldwide. Resistance to fluoroquinolones can, however, evolve rapidly, even during a course of treatment and numerous pathogens, including *Escherichia coli,* commonly exhibit resistance.

Surprisingly and of huge importance to the fight against antimicrobial resistance in the treatment of urinary tract infections, the Applicant has discovered that the antiretroviral drug zidovudine has a synergistic effect with fluoroquinolone antibiotics. In other words, the combination(s) has a greater biological activity than the expected additive effect of each agent at the stated dosage level.

Zidovudine (AZT) is a nucleoside analogue reverse-transcriptase inhibitor, a type of antiretroviral drug which is used for the treatment of HIV/AIDS infection. As well as its antiretroviral activity, the antibacterial effect of zidovudine (AZT) has been demonstrated both *in vitro* and *in vivo* with experimental models of gram-negative bacteria infections (Hermann et al., Antimicrob Agents Chemther. 1992 May; 36(5): 1081-1085). There have also been reports of zidovudine being active as an anti-microbial when combined with the antibiotic gentamicin (Doléans-Jordheim A. et al., Eur J Clin Microbiol Infect Dis. 2011 Oct;30(10):1249-56).

WO2014/147405 describes the use of zidovudine in combination with a polymyxin selected from colistin and polymyxin B for treating a microbial infection. WO2015/114340 describes the use of zidovudine in combination with a polymyxin selected from colistin or polymyxin B, an anti-tuberculosis antibiotic selected from rifampicin, rifapentine or rifabutin and optionally piperine, for treating a microbial infection. WO2018/011562 describes a combination comprising zidovudine and a carbapenem, optionally with a polymyxin selected from polymyxin B and polymyxin E.

Synergy is not, however, predictable or expected when two actives are used in combination. The present invention is therefore based on the unexpected finding that zidovudine or a pharmaceutically acceptable derivative thereof exhibits synergistic antimicrobial activity when used in combination with a fluoroquinolone antibiotic or a pharmaceutically acceptable derivative or prodrug thereof, against log phase (i.e. multiplying) microorganisms. Notably synergy is seen when the combinations are used against gram-negative bacteria.

The surprising biological activity of the combinations of the present invention offers the opportunity to rejuvenate certain urinary tract antibiotics, against which bacterial resistance has developed.

Synergy in the context of antimicrobial drugs is measured in a number of ways that conform to the generally accepted opinion that "synergy is an effect greater than additive". One of the ways to assess whether synergy has been observed is to use the "chequerboard" technique. This is a well-accepted method that leads to the generation of a value called the fractional inhibitory concentration index (FICI). Orhan et al., J. Clin. Microbiol. 2005, 43(1):140 describes the chequerboard method and analysis in the paragraph bridging pages 140-141, and explains that the FICI value is a ratio of the sum of the MIC (Minimum Inhibitory Concentration) level of each individual component alone and in the mixture. The combination is considered synergistic when the ΣFIC is ≤0.5, indifferent when the ΣFIC is >0.5 but <4.0, and antagonistic when the ΣFIC is >4.0.

Another accepted test for ascertaining the presence or absence of synergy is to use time-kill methods. This involves the dynamic effect of a drug combination being compared to each drug alone when assessing the effect on bacterial log or stationary-growth over time. Again, the possible results are for synergistic, additive or antagonistic effects.

### Summary of the Invention

In one aspect the present invention provides a combination of zidovudine or a pharmaceutically acceptable derivative thereof and a fluoroquinolone antibiotic or a pharmaceutically acceptable derivative or prodrug thereof, for use in the treatment of a microbial infection. In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

In another aspect the present invention provides the use of zidovudine or a pharmaceutically acceptable derivative thereof in combination with a fluoroquinolone antibiotic or a pharmaceutically acceptable derivative or prodrug thereof, in the manufacture of a medicament for treating a microbial infection. In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

In another aspect the present invention provides the use of a fluoroquinolone antibiotic or a pharmaceutically acceptable derivative or prodrug thereof, in combination with zidovudine or a pharmaceutically acceptable derivative thereof, in the manufacture of a medicament for treating a microbial infection. In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

In a further aspect, the invention provides a method of treating a microbial infection which comprises administering to a mammal, including man, zidovudine or a pharmaceutically acceptable derivative thereof in combination with a fluoroquinolone antibiotic or a pharmaceutically acceptable derivative or prodrug thereof. In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

There is also provided a pharmaceutical composition comprising zidovudine or a pharmaceutically acceptable derivative thereof in combination with a fluoroquinolone antibiotic or a pharmaceutically acceptable derivative or prodrug thereof, and a pharmaceutically acceptable adjuvant, diluent or carrier, for use in the treatment of a microbial infection, preferably wherein the microbial infection is a bacterial infection, e.g. a gram-negative bacterial infection. In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

In a further aspect, the invention relates to a product comprising zidovudine or a pharmaceutically acceptable derivative thereof and a fluoroquinolone antibiotic or a pharmaceutically acceptable derivative or prodrug thereof, as a combined preparation for simultaneous, separate or sequential use in killing multiplying microorganisms associated with a microbial infection. Preferably for killing multiplying bacteria associated with a bacterial infection, e.g. a gram-negative bacterial infection. In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

### Brief Description of the Figures

Having thus described aspects of the disclosure in the foregoing general terms, reference will now be made to the accompanying drawings, which are not necessarily drawn to scale. The drawings are exemplary only, and should not be construed as limiting the disclosure.

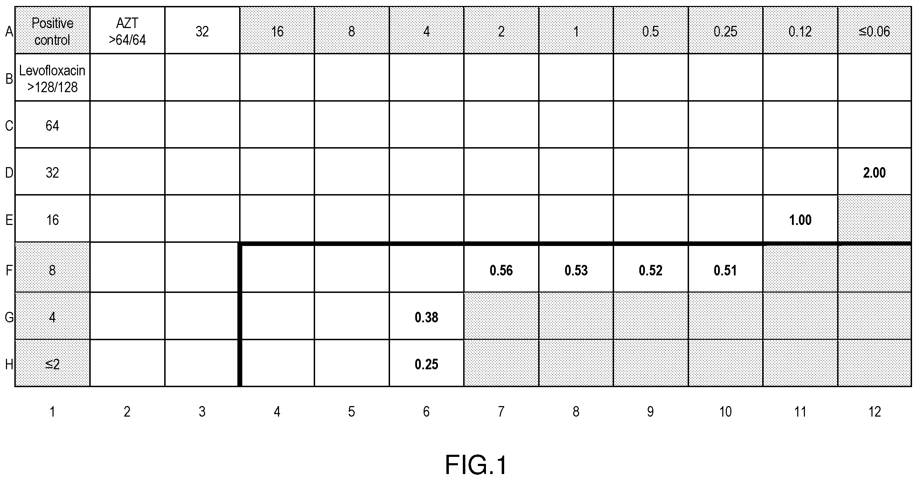

Figure 1 is a chequerboard from Example 4 showing synergy testing of AZT and levofloxacin using the broth microdilution checkerboard method for Escherichia coli using the 100% reading criterion. Shading indicates growth wells; thick border indicates area of expected growth. Compound concentrations (in mg/L) are shown in wells of the top row and left column; ΣFIC values are shown in bold. ΣFICmin: 0.25 (synergy) at H6. ΣFICmax: 2.00 (indifference) at D12.

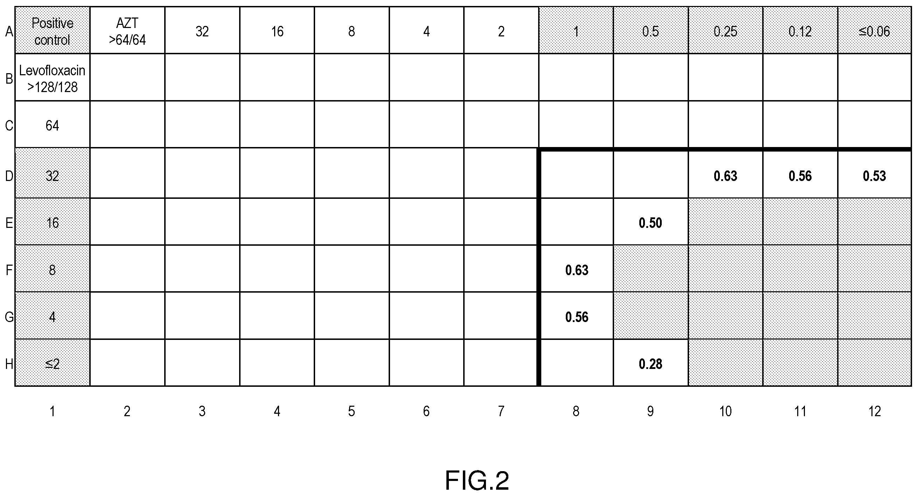

Figure 2 is a chequerboard from Example 4 showing synergy testing of AZT and levofloxacin using the broth microdilution checkerboard method for Escherichia coli using the 100% reading criterion. Shading indicates growth wells; thick border indicates area of expected growth. Compound concentrations (in mg/L) are shown in wells of the top row and left column; ΣFIC values are shown in bold. ΣFICmin: 0.28 (synergy) at H9. ΣFICmax: 0.63 at D10 and F8.

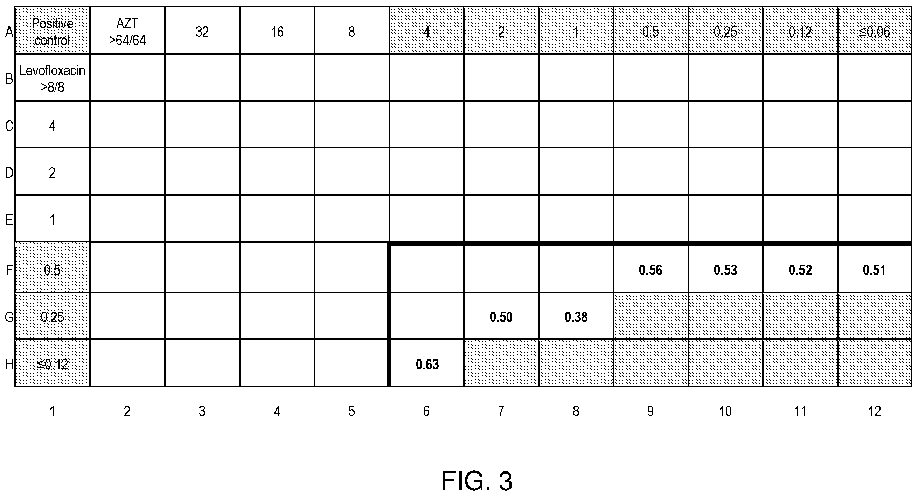

Figure 3 is a chequerboard from Example 4 showing synergy testing of AZT and levofloxacin using the broth microdilution checkerboard method for Klebsiella using the 100% reading criterion. Shading indicates growth wells; thick border indicates area of expected growth. Compound concentrations (in mg/L) are shown in wells of the top row and left column; ΣFIC values are shown in bold. ΣFICmin: 0.38 (synergy) at G8. ΣFICmax: 0.63 at H6.

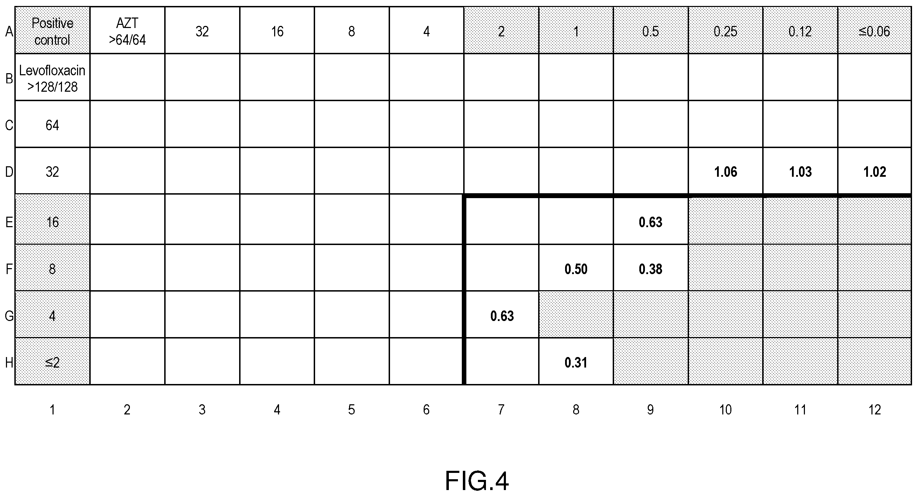

Figure 4 is a chequerboard from Example 4 showing synergy testing of AZT and levofloxacin using the broth microdilution checkerboard method for *Klebsiella pneumoniae* using the 100% reading criterion. Shading indicates growth wells; thick border indicates area of expected growth. Compound concentrations (in mg/L) are shown in wells of the top row and left column; ΣFIC values are shown in bold. ΣFICmin: 0.31 (synergy) at H8. ΣFICₘₐₓ: 1.06 at D10.

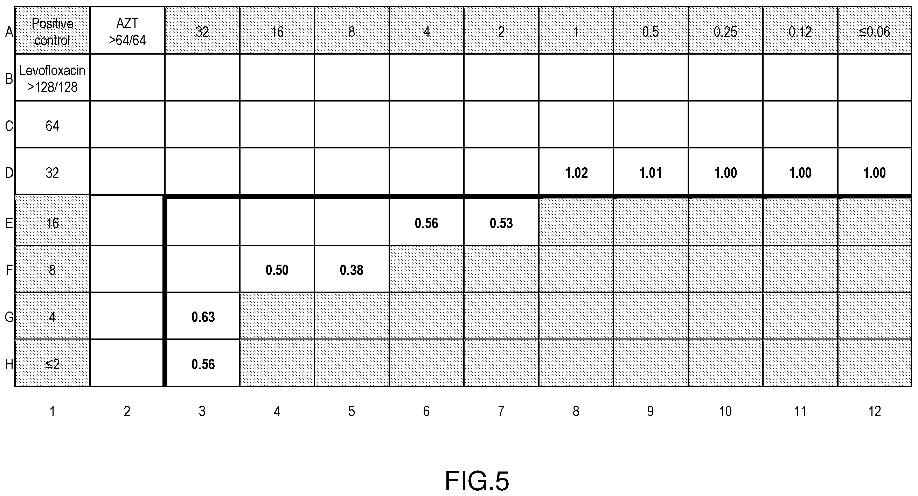

Figure 5 is a chequerboard from Example 4 showing synergy testing of AZT and levofloxacin using the broth microdilution checkerboard method for *Klebsiella pneumoniae* using the 100% reading criterion. Shading indicates growth wells; thick border indicates area of expected growth. Compound concentrations (in mg/L) are shown in wells of the top row and left column; ΣFIC values are shown in bold. ΣFICₘᵢₙ: 0.38 (synergy) at F5. ΣFICₘₐₓ: 1.02 (indifference) at D8.

### Detailed Description of the Invention

As used herein, the expressions *"combination of'* and *"in combination with"* cover separate, sequential and simultaneous administration of the agents. Unless specified to the contrary, the expressions are also intended to exclude any additional actives, e.g. "a combination of zidovudine and ciprofloxacin" means that zidovudine and ciprofloxacin are administered separately, sequentially or simultaneously but that no other actives are administered.

When the agents are administered sequentially, either the zidovudine or the fluoroquinolone antibiotic compound may be administered first. When administration is simultaneous, the agents may be administered either in the same or a different pharmaceutical composition. Adjunctive therapy, i.e. where one agent is used as a primary treatment and the other agent(s) is used to assist that primary treatment, is also an embodiment of the present invention.

The combinations of the present invention may be used to treat microbial infections. In particular they may be used to kill multiplying and/or clinically latent microorganisms associated with microbial infections, preferably multiplying microorganisms associated with microbial infections, e.g. multiplying bacteria associated with Gram-negative bacterial infections. References herein to the treatment of a microbial infection therefore include killing multiplying and/or clinically latent microorganisms associated with such infections.

As used herein, *"kill"* means a loss of viability as assessed by a lack of metabolic activity.

As used herein, *"clinically latent microorganism"* means a microorganism that is metabolically active but has a growth rate that is below the threshold of infectious disease expression. The threshold of infectious disease expression refers to the growth rate threshold below which symptoms of infectious disease in a host are absent.

The metabolic activity of clinically latent microorganisms can be determined by several methods known to those skilled in the art; for example, by measuring mRNA levels in the microorganisms or by determining their rate of uridine uptake. In this respect, clinically latent microorganisms, when compared to microorganisms under logarithmic growth conditions (*in vitro* or *in vivo*), possess reduced but still significant levels of:
(I) mRNA (e.g. from 0.0001 to 50%, such as from 1 to 30, 5 to 25 or 10 to 20%, of the level of mRNA); and/or
(II) uridine (e.g. [³H]uridine) uptake (e.g. from 0.0005 to 50%, such as from 1 to 40, 15 to 35 or 20 to 30% of the level of [³H]uridine uptake).

Clinically latent microorganisms typically possess a number of identifiable characteristics. For example, they may be viable but non-culturable; i.e. they cannot typically be detected by standard culture techniques, but are detectable and quantifiable by techniques such as broth dilution counting, microscopy, or molecular techniques such as polymerase chain reaction. In addition, clinically latent microorganisms are phenotypically tolerant, and as such are sensitive (in log phase) to the biostatic effects of conventional antimicrobial agents (i.e. microorganisms for which the minimum inhibitory concentration (MIC) of a conventional antimicrobial is substantially unchanged); but possess drastically decreased susceptibility to drug-induced killing (e.g. microorganisms for which, with any given conventional antimicrobial agent, the ratio of minimum microbiocidal concentration (e.g. minimum bactericidal concentration, MBC) to MIC is 10 or more).

As used herein, the term *"microorganisms"* means fungi and bacteria. References herein to *"microbia", "antimicrobial"* and *"antimicrobially"* shall be interpreted accordingly. For example, the term *"microbial"* means fungal or bacterial, and *"microbial infection"* means any fungal or bacterial infection.

In various embodiments of the invention, one or more of the aforementioned combinations is used to treat a bacterial infection, in particular the combinations may be used to kill multiplying and/or clinically latent microorganisms associated with a bacterial infection. Preferably multiplying bacteria associated with a bacterial infection. As used herein, the term *"bacteria"* (and derivatives thereof, such as *"microbial infection*") includes, but is not limited to, references to organisms (or infections due to organisms) of the following classes and specific types:
Gram-positive cocci, such as Staphylococci (e.g. *Staph. aureus, Staph. epidermidis, Staph. saprophyticus, Staph. auricularis, Staph. capitis capitis, Staph. c. ureolyticus, Staph. caprae, Staph. cohnii cohnii, Staph. c. urealyticus, Staph. equorum, Staph. gallinarum, Staph. haemolyticus, Staph. hominis hominis, Staph. h. novobiosepticius, Staph. hyicus, Staph. intermedius, Staph. lugdunensis, Staph. pasteuri, Staph. saccharolyticus, Staph. schleiferi schleiferi, Staph. s. coagulans, Staph. sciuri, Staph. simulans, Staph. warneri* and *Staph. xylosus*); Streptococci (e.g.beta-haemolytic, pyogenic streptococci (such as *Strept. agalactiae, Strept. canis, Strept. dysgalactiae dysgalactiae, Strept. dysgalactiae equisimilis, Strept. equi equi, Strept. equi zooepidemicus, Strept. iniae, Strept. porcinus* and *Strept. pyogenes*), microaerophilic, pyogenic streptococci (Streptococcus "milleri", such as *Strept. anginosus, Strept. constellatus constellatus, Strept. constellatus pharyngidis* and *Strept. intermedius*), oral streptococci of the "mitis" (alpha-haemolytic - Streptococcus "viridans", such as *Strept. mitis, Strept. oralis, Strept. sanguinis, Strept. cristatus, Strept. gordonii* and *Strept. parasanguinis*), "salivarius" (non-haemolytic, such as *Strept. salivarius* and *Strept. vestibularis*) and "mutans" (tooth-surface streptococci, such as *Strept. criceti, Strept. mutans, Strept. ratti* and *Strept. sobrinus*) groups, *Strept. acidominimus, Strept. bovis, Strept. faecalis, Strept. equinus, Strept. pneumoniae* and *Strept. suis,* or Streptococci alternatively classified as Group A, B, C, D, E, G, L, P, U or V Streptococcus);
Gram-negative cocci, such as *Neisseria gonorrhoeae, Neisseria meningitidis, Neisseria cinerea, Neisseria elongata, Neisseria flavescens, Neisseria lactamica, Neisseria mucosa, Neisseria sicca, Neisseria subflava* and *Neisseria weaveri;* Bacillaceae, such as *Bacillus anthracis, Bacillus subtilis, Bacillus thuringiensis, Bacillus stearothermophilus* and *Bacillus cereus*; Enterobacteriaceae, such as *Escherichia coli,* Enterobacter (e.g. *Enterobacter aerogenes, Enterobacter agglomerans* and *Enterobacter cloacae*), Citrobacter (such as *Citrob. freundii* and *Citrob. divernis*), Hafnia (e.g. *Hafnia alvei*)*,* Erwinia (e.g. *Erwinia persicinus*), *Morganella morganii,* Salmonella (*Salmonella enterica* and *Salmonella typhi*), Shigella (e.g. *Shigella dysenteriae, Shigella flexneri, Shigella boydii* and *Shigella sonnei*)*,* Klebsiella (e.g. *Klebs. pneumoniae, Klebs. oxytoca, Klebs. ornitholytica, Klebs. planticola, Klebs. ozaenae, Klebs. terrigena, Klebs. granulomatis (Calymmatobacterium granulomatis)* and *Klebs. rhinoscleromatis*), Proteus (e.g. *Pr. mirabilis, Pr. rettgeri* and *Pr. vulgaris*), Providencia (e.g. *Providencia alcalifaciens, Providencia rettgeri* and *Providencia stuartii*)*,* Serratia (e.g. *Serratia marcescens* and *Serratia liquifaciens),* and Yersinia (e.g. *Yersinia enterocolitica, Yersinia pestis* and *Yersinia pseudotuberculosis*); Enterococci (e.g. *Enterococcus avium, Enterococcus casseliflavus, Enterococcus cecorum, Enterococcus dispar, Enterococcus durans, Enterococcus faecalis, Enterococcus faecium, Enterococcus flavescens, Enterococcus gallinarum, Enterococcus hirae, Enterococcus malodoratus, Enterococcus mundtii, Enterococcus pseudoavium, Enterococcus raffinosus* and *Enterococcus solitarius*); Helicobacter (e.g. *Helicobacter pylori, Helicobacter cinaedi* and *Helicobacter fennelliae*); Acinetobacter (e.g. *A. baumanii, A. calcoaceticus, A. haemolyticus, A. johnsonii, A. junii, A. Iwoffi* and A. *radioresistens*); Pseudomonas (e.g. *Ps. aeruginosa, Ps. maltophilia* (*Stenotrophomonas maltophilia), Ps. alcaligenes, Ps. chlororaphis, Ps. fluorescens, Ps. luteola. Ps. mendocina, Ps. monteilii, Ps. oryzihabitans, Ps. pertocinogena, Ps. pseudalcaligenes, Ps. putida* and *Ps. stutzeri*); *Bacteriodes fragilis*; Peptococcus (e.g. *Peptococcus niger);* Peptostreptococcus; Clostridium (e.g. *C. perfringens, C. difficile, C. botulinum, C. tetani, C. absonum, C. argentinense, C. baratii, C. bifermentans, C. beijerinckii, C. butyricum, C. cadaveris, C. carnis, C. celatum, C. clostridioforme, C. cochlearium, C. cocleatum, C. fallax, C. ghonii, C. glycolicum, C. haemolyticum, C. hastiforme, C. histolyticum, C. indolis, C. innocuum, C. irregulare, C. leptum, C. limosum, C. malenominatum, C. novyi, C. oroticum, C. paraputrificum, C. piliforme, C. putrefasciens, C. ramosum, C. septicum, C. sordelii, C. sphenoides, C. sporogenes, C. subterminale, C. symbiosum* and *C. tertium*); Mycoplasma (e.g. *M. pneumoniae, M. hominis, M. genitalium* and *M*. *urealyticum*); Mycobacteria (e.g. *Mycobacterium tuberculosis, Mycobacterium avium, Mycobacterium fortuitum, Mycobacterium marinum, Mycobacterium kansasii, Mycobacterium chelonae, Mycobacterium abscessus, Mycobacterium leprae, Mycobacterium smegmitis, Mycobacterium africanum, Mycobacterium alvei, Mycobacterium asiaticum, Mycobacterium aurum, Mycobacterium bohemicum, Mycobacterium bovis, Mycobacterium branderi, Mycobacterium brumae, Mycobacterium celatum, Mycobacterium chubense, Mycobacterium confluentis, Mycobacterium conspicuum, Mycobacterium cookii, Mycobacterium flavescens, Mycobacterium gadium, Mycobacterium gastri, Mycobacterium genavense, Mycobacterium gordonae, Mycobacterium goodii, Mycobacterium haemophilum, Mycobacterium hassicum, Mycobacterium intracellulare, Mycobacterium interjectum, Mycobacterium heidelberense, Mycobacterium lentiflavum, Mycobacterium malmoense, Mycobacterium microgenicum, Mycobacterium microti, Mycobacterium mucogenicum, Mycobacterium neoaurum, Mycobacterium nonchromogenicum, Mycobacterium peregrinum, Mycobacterium phlei, Mycobacterium scrofulaceum, Mycobacterium shimoidei, Mycobacterium simiae, Mycobacterium szulgai, Mycobacterium terrae, Mycobacterium thermoresistabile, Mycobacterium triplex, Mycobacterium triviale, Mycobacterium tusciae, Mycobacterium ulcerans, Mycobacterium vaccae, Mycobacterium wolinskyi* and *Mycobacterium xenopi*); Haemophilus (e.g. *Haemophilus influenzae, Haemophilus ducreyi, Haemophilus aegyptius, Haemophilus parainfluenzae, Haemophilus haemolyticus* and *Haemophilus parahaemolyticus*); Actinobacillus (e.g. *Actinobacillus actinomycetemcomitans, Actinobacillus equuli, Actinobacillus hominis, Actinobacillus lignieresii, Actinobacillus suis* and *Actinobacillus ureae); Actinomyces* (e.g. *Actinomyces israelii*); Brucella (e.g. *Brucella abortus, Brucella canis, Brucella melintensis* and *Brucella suis*); Campylobacter (e.g. *Campylobacter jejuni, Campylobacter coli, Campylobacter lari* and *Campylobacter fetus*); *Listeria monocytogenes*; Vibrio (e.g. *Vibrio cholerae* and *Vibrio parahaemolyticus, Vibrio alginolyticus, Vibrio carchariae, Vibrio fluvialis, Vibrio furnissii, Vibrio hollisae, Vibrio metschnikovii, Vibrio mimicus* and *Vibrio vulnificus*); *Erysipelothrix rhusopathiae*; Corynebacteriaceae (e.g. *Corynebacterium diphtheriae, Corynebacterium jeikeum* and *Corynebacterium urealyticum*); Spirochaetaceae, such as Borrelia (e.g. *Borrelia recurrentis, Borrelia burgdorferi, Borrelia afzelii, Borrelia andersonii, Borrelia bissettii, Borrelia garinii, Borrelia japonica, Borrelia lusitaniae, Borrelia tanukii, Borrelia turdi, Borrelia valaisiana, Borrelia caucasica, Borrelia crocidurae, Borrelia duttoni, Borrelia graingeri, Borrelia hermsii, Borrelia hispanica, Borrelia latyschewii, Borrelia mazzottii, Borrelia parkeri, Borrelia persica, Borrelia turicatae* and *Borrelia venezuelensis*) and Treponema (*Treponema pallidum* ssp. *pallidum, Treponema pallidum* ssp. *endemicum, Treponema pallidum* ssp. *pertenue* and *Treponema carateum*); Pasteurella (e.g. *Pasteurella aerogenes, Pasteurella bettyae, Pasteurella canis, Pasteurella dagmatis, Pasteurella gallinarum, Pasteurella haemolytica, Pasteurella multocida multocida, Pasteurella multocida gallicida, Pasteurella multocida septica, Pasteurella pneumotropica* and *Pasteurella stomatis*); Bordetella (e.g. *Bordetella bronchiseptica, Bordetella hinzii, Bordetella holmseii, Bordetella parapertussis, Bordetella pertussis and Bordetella trematum);* Nocardiaceae, such as Nocardia (e.g. *Nocardia asteroides* and *Nocardia brasiliensis*); Rickettsia (e.g. *Ricksettsii* or *Coxiella burnetii*); Legionella (e.g. *Legionalla anisa, Legionalla birminghamensis, Legionalla bozemanii, Legionalla cincinnatiensis, Legionalla dumoffii, Legionalla feeleii, Legionalla gormanii, Legionalla hackeliae, Legionalla israelensis, Legionalla jordanis, Legionalla lansingensis, Legionalla longbeachae, Legionalla maceachernii, Legionalla micdadei, Legionalla oakridgensis, Legionalla pneumophila, Legionalla sainthelensi, Legionalla tucsonensis* and *Legionalla wadsworthii*); *Moraxella catarrhalis; Cyclospora cayetanensis; Entamoeba histolytica; Giardia lamblia; Trichomonas vaginalis; Toxoplasma gondii; Stenotrophomonas maltophilia; Burkholderia cepacia; Burkholderia mallei and Burkholderia pseudomallei*; *Francisella tularensis*; Gardnerella (e.g. *Gardneralla vaginalis* and *Gardneralla mobiluncus*); *Streptobacillus moniliformis*; Flavobacteriaceae, such as Capnocytophaga (e.g. *Capnocytophaga canimorsus, Capnocytophaga cynodegmi, Capnocytophaga gingivalis, Capnocytophaga granulosa, Capnocytophaga haemolytica, Capnocytophaga ochracea* and *Capnocytophaga sputigena*); Bartonella (*Bartonella bacilliformis, Bartonella clarridgeiae, Bartonella elizabethae, Bartonella henselae, Bartonella quintana* and *Bartonella vinsonii arupensis*); Leptospira (e.g. *Leptospira biflexa, Leptospira borgpetersenii, Leptospira inadai, Leptospira interrogans, Leptospira kirschneri, Leptospira noguchii, Leptospira santarosai* and *Leptospira weilii*); Spirillium (e.g. *Spirillum minus*); Baceteroides (e.g. *Bacteroides caccae, Bacteroides capillosus, Bacteroides coagulans, Bacteroides distasonis, Bacteroides eggerthii, Bacteroides forsythus, Bacteroides fragilis, Bacteroides merdae, Bacteroides ovatus, Bacteroides putredinis, Bacteroides pyogenes, Bacteroides splanchinicus, Bacteroides stercoris, Bacteroides tectus, Bacteroides thetaiotaomicron, Bacteroides uniformis, Bacteroides ureolyticus* and *Bacteroides vulgatus*); Prevotella (e.g. *Prevotella bivia, Prevotella buccae, Prevotella corporis, Prevotella dentalis (Mitsuokella dentalis), Prevotella denticola, Prevotella disiens, Prevotella enoeca, Prevotella heparinolytica, Prevotella intermedia, Prevotella loeschii, Prevotella melaninogenica, Prevotella nigrescens, Prevotella oralis, Prevotella oris, Prevotella oulora, Prevotella tannerae, Prevotella venoralis* and *Prevotella zoogleoformans*); Porphyromonas (e.g. *Porphyromonas asaccharolytica, Porphyromonas cangingivalis, Porphyromonas canoris, Porphyromonas cansulci, Porphyromonas catoniae, Porphyromonas circumdentaria, Porphyromonas crevioricanis, Porphyromonas endodontalis, Porphyromonas gingivalis, Porphyromonas gingivicanis, Porphyromonas levii* and *Porphyromonas macacae*); Fusobacterium (e.g. *F. gonadiaformans, F. mortiferum, F. naviforme, F. necrogenes, F. necrophorum necrophorum, F. necrophorum fundiliforme, F. nucleatum nucleatum, F. nucleatum fusiforme, F. nucleatum polymorphum, F. nucleatum vincentii, F. periodonticum, F. russii, F. ulcerans* and *F. varium*); Chlamydia (e.g. *Chlamydia trachomatis*); Cryptosporidium (e.g. *C. parvum, C. hominis, C. canis, C. felis, C. meleagridis* and *C. muris*); Chlamydophila (e.g. *Chlamydophila abortus* (*Chlamydia psittaci*), *Chlamydophila pneumoniae* (*Chlamydia pneumoniae*) and *Chlamydophila psittaci* (*Chlamydia psittaci*)); Leuconostoc (e.g. *Leuconostoc citreum, Leuconostoc cremoris, Leuconostoc dextranicum, Leuconostoc lactis, Leuconostoc mesenteroides* and *Leuconostoc pseudomesenteroides*); Gemella (e.g. *Gemella bergeri, Gemella haemolysans, Gemella morbillorum* and *Gemella sanguinis*); and Ureaplasma (e.g. *Ureaplasma parvum* and *Ureaplasma urealyticum*).

Preferably, the bacterial infections treated by the combinations described herein are Gram-negative bacterial infections. Particular Gram-negative bacteria that may be treated using a combination of the invention include:
Enterobacteriaceae, such as *Escherichia coli,* Klebsiella (e.g. *Klebs. pneumoniae* and *Klebs. oxytoca*) and Proteus (e.g. *Pr. mirabilis, Pr. rettgeri* and *Pr. vulgaris*); *Haemophilis influenzae*; Mycobacteria, such as *Mycobacterium tuberculosis*; and *Enterobacter* (e.g. *Enterobacter cloacae*). Preferably, the bacteria are Enterobacteriaceae, such as *Escherichia coli* and Klebsiella (e.g. *Klebs. pneumoniae* and *Klebs. oxytoca*). Particularly preferred are *Escherichia coli,* and *Klebs. pneumoniae* (e.g. *Klebs. pneumoniae* subsp. *pneumoniae*).

In all embodiments it is preferable that the combination therapy is synergistic as compared to the administration of the combination components taken alone.

The combination of the present invention is particularly beneficial in treating (multi)-drug-resistant ((M)DR) bacteria. With respect to Enterobacteriaceae, drug resistance most often builds up to carbapenemase i.e. carbapenemase-resistant strains and "extended spectrum β-lactamase" (ESBL) strains for example New Delhi Metallo-beta-lactamase-1 (NDM-1) resistant *Klebs. Pneumonia,* and NDM-1 *E.coli.*

It should be kept in mind that although a combination such as that claimed may initially be demonstrated to be functional in treating (M)DR strains, they can then be used in treating non-resistant strains. This is especially valuable in the context of the presently claimed combination where the primary therapy for Enterobacteriaceae, such as *Escherichia coli,* and Klebsiella (e.g. *Klebs. pneumoniae* and *Klebs. oxytoca*) are antimicrobial drugs that are expensive due to prevailing patent protection. The replacement of such "ethical" drugs by a combination of "generic" antibiotics is thought to be beneficial from a therapeutic perspective as well as financial/economic perspective in times where governments are seeking to reduce the cost of healthcare.

The combinations of the present invention may be used to treat infections associated with any of the above-mentioned bacterial organisms, and in particular they may be used for killing multiplying and/or clinically latent microorganisms associated with such an infection, e.g. a Gram-negative bacterial infection.

Particular conditions which may be treated using the combination of the present invention include those which are caused by Gram-negative bacteria such as abscesses, asthma, bacilliary dysentry, bacterial conjunctivitis, bacterial keratitis, bacterial vaginosis, bone and joint infections, bronchitis (acute or chronic), brucellosis, burn wounds, cat scratch fever, cellulitis, chancroid, cholangitis, cholecystitis, cystic fibrosis, cystitis, nephritis, diffuse panbronchiolitis, dental caries, diseases of the upper respiratory tract, empymea, endocarditis, endometritis, enteric fever, enteritis, epididymitis, epiglottitis, eye infections, furuncles, gardnerella vaginitis, gastrointestinal infections (gastroenteritis), genital infections, gingivitis, gonorrhoea, granuloma inguinale, Haverhill fever, infected burns, infections following dental operations, infections in the oral region, infections associated with prostheses, intraabdominal abscesses, Legionnaire's disease, leptospirosis, listeriosis, liver abscesses, Lyme disease, lymphogranuloma venerium, mastitis, mastoiditis, meningitis and infections of the nervous system, non-specific urethritis, opthalmia (e.g. opthalmia neonatorum), osteomyelitis, otitis (e.g. otitis externa and otitis media), orchitis, pancreatitis, paronychia, pelveoperitonitis, peritonitis, peritonitis with appendicitis, pharyngitis, pleural effusion, pneumonia, postoperative wound infections, postoperative gas gangrene, prostatitis, pseudo-membranous colitis, psittacosis, pyelonephritis, Q fever, rat-bite fever, Ritter's disease, salmonellosis, salpingitis, septic arthritis, septic infections, septicameia, systemic infections, tonsillitis, trachoma, typhoid, urethritis, urinary tract infections, wound infections; or infections with, *Escherichia coli, Klebs. pneumoniae, Klebs. oxytoca, Pr. mirabilis, Pr. rettgeri, Pr. vulgaris, Haemophilis influenzae, Enterococcus faecalis, Enterococcus faecium,* and *Enterobacter cloacae.*

In one embodiment the combinations of the invention are used to treat urinary tract infections.

It will be appreciated that references herein to *"treatment"* extend to prophylaxis as well as the treatment of established diseases or symptoms.

In various embodiments, one or more of the herein described combinations is used for treating a microbial infection in a subject other than an HIV-infected subject. HIV refers to the "human immunodeficiency virus". By the expression "other than an HIV-infected subject" means that the subject being treated has not been diagnosed with HIV and is not infected with HIV; the subject is "HIV-negative". The purpose of this disclaimer is to exclude the use of the fluoroquinolone antibiotic to treat a bacterial infection in a HIV-infected subject who is receiving AZT as part of their management of HIV. The present invention differs from such use in that it is the combination of AZT and a fluoroquinolone which is used to treat the same microbial, e.g. bacterial infection.

As used herein the term *"pharmaceutically acceptable derivative"* means: (a) pharmaceutically acceptable salts; and/or (b) solvates (including hydrates). Pharmaceutically acceptable salts of the compounds included in the combinations of the invention include suitable acid addition or base salts thereof. A review of suitable pharmaceutical salts may be found in Berge et al, J Pharm Sci, 66, 1-19 (1977).

Suitable acid addition salts include carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxybenzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulfonate salts (e.g. benzenesulfonate, methyl-, bromo- or chloro-benzenesulfonate, xylenesulfonate, methanesulfonate, ethanesulfonate, propanesulfonate, hydroxyethanesulfonate, 1- or 2-naphthalene-sulfonate or 1,5-naphthalenedisulfonate salts) or sulfate, pyrosulfate, bisulfate, sulfite, bisulfite, phosphate, monohydrogenphosphate, dihydrogenphosphate, metaphosphate, pyrophosphate or nitrate salts. Suitable base salts include metal salts, e.g. sodium, calcium, and amine salts

For example, ciprofloxacin hydrochloride, ciprofloxacin hydrochloride hydrate, ciprofloxacin formamide, ciprofloxacin lactate, gatifloxacin sesquihydrate, gemifloxacin mesylate, levofloxacin hemihydrate, moxifloxacin hydrochloride, lomefloxacin hydrochloride, pefloxacin mesylate dihydrate, or besifloxacin hydrochloride.

As used herein the term *"prodrug"* means the antimicrobial compound, wherein one or more groups have been modified such that the modification may be reversed upon administration to a human or mammalian subject. Such reversion is usually performed by an enzyme naturally present in such subject, though it is possible for a second agent to be administered together with such a prodrug in order to perform the reversion in vivo. Examples of such modifications include ester formation (for example, any of those described above), wherein the reversion may be carried out be an esterase etc. Other such systems will be well known to those skilled in the art. Zidovudine is, for example, a prodrug that must be phosphorylated to its active 5'-triphosphate metabolite.

The invention includes the use of these pharmaceutically acceptable derivatives and prodrugs.

The invention also includes where appropriate all enantiomers and tautomers of the compounds. The skilled person will recognise compounds that possess optical properties (one or more chiral carbon atoms) or tautomeric characteristics. The corresponding enantiomers and/or tautomers may be isolated or prepared by methods known in the art.

Some of the compounds included in the combinations of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the compounds or pharmaceutically acceptable salts thereof. An isotopic variation or a pharmaceutically acceptable salt thereof is defined as one in which at least one atom is replaced by an atom having the same atomic number but an atomic mass different from the atomic mass usually found in nature. Examples of isotopes that can be incorporated include isotopes of hydrogen, carbon, nitrogen, oxygen, phosphorus, sulphur, fluorine and chlorine such as 2H, 3H, 13C, 14C, 15N, 170, 180, 31P, 32P, 35S, 18F and 36CI, respectively. Certain isotopic variations, for example, those in which a radioactive isotope such as 3H or 14C is incorporated, are useful in drug and/or substrate tissue distribution studies. Tritiated, i.e., 3H, and carbon-14, i.e., 14C, isotopes are particularly preferred for their ease of preparation and detectability. Further, substitution with isotopes such as deuterium, i.e., 2H, may afford certain therapeutic advantages resulting from greater metabolic stability, for example, increased in vivo half-life or reduced dosage requirements and hence may be preferred in some circumstances. Isotopic variations can generally be prepared by conventional procedures using appropriate isotopic variations of suitable reagents.

The compounds for use in the combination of the present invention, including the pharmaceutically acceptable derivatives or prodrugs thereof, are commercially available and/or can be prepared by synthesis methods known in the art. Zidovudine, ciprofloxacin, ciprofloxacin hydrochloride, ciprofloxacin hydrochloride hydrate, ciprofloxacin formamide, ciprofloxacin lactate, gatifloxacin, gatifloxacin sesquihydrate, gemifloxacin, gemifloxacin mesylate, levofloxacin, levofloxacin hemihydrate, moxifloxacin, moxifloxacin hydrochloride, lomefloxacin, lomefloxacin hydrochloride, norfloxacin, ofloxacin, pefloxacin, pefloxacin mesylate dihydrate, balofloxacin, grepafloxacin, pazufloxacin, sparfloxacin, sitafloxacin, besifloxacin, besifloxacin hydrochloride, and delafloxacin are for example available from Sigma-Aldrich^{®}.

Other commercial suppliers are known in the art.

Zidovudine is 1-[(2*R*, 4*S,* 5*S*)-4-Azido-5-(hydroxymethyl)oxolan-2-yl]-5-methylpyrimidine-2,4-dione, and is available by prescription under the trade name Retrovir^{®}. It is also known as 3'-azido-3'-deoxythymidine or "AZT" and has the following chemical structure:

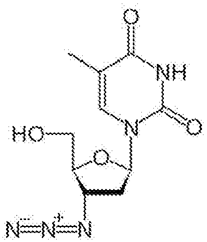

Ciprofloxacin is an antibiotic used to treat a number of bacterial infections; it is a second-generation fluoroquinolone. This includes bone and joint infections, intra abdominal infections, certain types of infectious diarrhoea, respiratory tract infections, skin infections, typhoid fever, and urinary tract infections, among others. It is also known as 1-cyclopropyl-6-fluoro-1,4-dihydro-4-oxo-7-(1-piperazinyl)-3-quinolinecarboxylic acid, and as well as being commercially available, ciprofloxacin is sold as a generic medication and under various trade names including Cetraxal, Cilodex, Ciloxan, Cipro and Neofloxin. Ciprofloxacin has the following chemical structure:

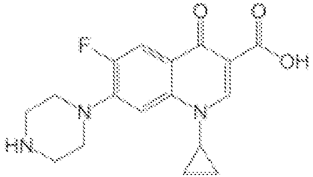

Gatifloxacin is an antibiotic of the fourth-generation fluoroquinolone family, which is currently only available in the US and Canada as an ophthalmic solution. Its chemical name is (±)-1-cyclopropyl-6-fluoro-1,4-dihydro-8-methoxy-7(3-methyl-1-piperazinyl)-4-oxo-3-quinolinecarboxylic acid, anhydrous. Gatifloxacin is associated with the brand names Gatiflo, Tequin and Zymar, and has the following chemical structure:

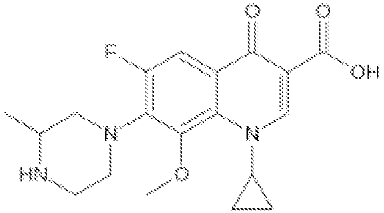

Gemifloxacin is most commonly used as its mesylate salt; gemifloxacin mesylate is an oral broad-spectrum fourth-generation fluoroquinolone antibacterial agent used in the treatment of acute bacterial exacerbation of chronic bronchitis and mild-to-moderate pneumonia. Gemifloxacin mesylate is commercially available and known under the trade name Factive. Gemifloxacin has the chemical name 7-[(4Z)-3-aminomethyl)-4-methoxyiminopyrrolidin-1-yl]-1-cyclopropyl-6-fluoro-4-oxo-1,8-naphthyridine-3-carboxylic acid and the following chemical structure:

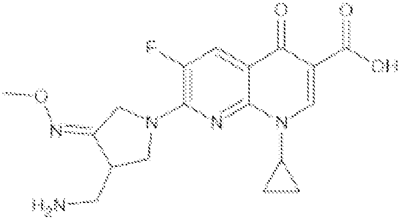

Levofloxacin is a third-generation fluoroquinolone antibiotic which is used to treat a number of bacterial infections including acute bacterial sinusitis, pneumonia, urinary tract infections, chronic prostatitis, and some types of gastroenteritis. It is the "left-sided" or levo isomer of the racemate ofloxacin. It is therefore a chiral fluoroquinolone and the pure (-)-(*S*)-enantiomer of the racemic ofloxacin.

Levofloxacin is available commercially and marketed under the trade names Levaquin, Tavanic, Iquix, amongst others. Its chemical name is (2S)-7-fluoro-2-methyl-6-(4-methylpiperazin-1-yl)-10-oxo-4-oxa-1-azatricyclo[7.3.1.0^{5,13}]trideca-5(13),6,8,11-tetraene-11-carboxylic acid. The chemical structure of levofloxacin is:

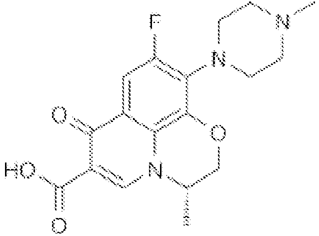

Moxifloxacin is an antibiotic used to treat a number of bacterial infections including pneumonia, conjunctivitis, endocarditis, tuberculosis, and sinusitis. It is a fourth-generation fluoroquinolone and sold under the trade names Avelox, Vigamox, and Moxiflox, among others. Its chemical name is 7-[(4aS,7aS)-1,2,3,4,4a,5,7,7a-octahydropyrrolo[3,4-b]pyridin-6-yl]-1-cyclopropyl-6-fluoro-8-methoxy-4-oxoquinoline-3-carboxylic acid, and has the following chemical structure:

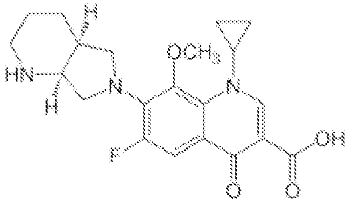

Lomefloxacin is sold as lomefloxacin hydrochloride under the brand names Maxaquin, Okacyn and Uniquin. It is a fluoroquinolone antibiotic used to treat bacterial infections including bronchitis and urinary tract infections. The chemical name of lomefloxacin is 1-ethyl-6,8-difluoro-7-(3-methylpiperazin-1-yl)-4-oxoquinoline-3-carboxylic acid and it has the following structure:

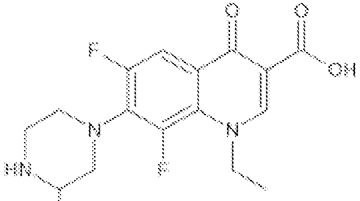

Norfloxacin is a first-genearation fluoroquinolone used to treat urinary tract infections, gynaecological infections, inflammation of the prostate gland, gonorrhoea and bladder infections. It is sold under the brand name Noroxin, among others, has the chemical name 1-ethyl-6-fluoro-4-oxo-7-piperazin-1-ylquinoline-3-carboxylic acid and the chemical structure:

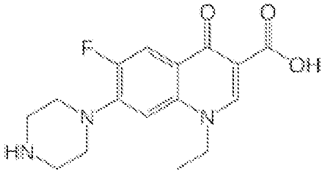

Ofloxacin is a second-generation fluoroquinolone and a broader spectrum analog of norfloxacin. It is active against both Gram-positive and Gram-negative bacteria and is sold under the brand names of Floxin and Ocuflox, among others. Its chemical name is 7-fluoro-2-methyl-6-(4-methylpiperazin-1-yl)-10-oxo-4-oxa-1-azatricyclo[7.3.1.0^{5,13}]trideca-5(13),6,8,11-tetraene-11-carboxylic acid and its chemical structure is:

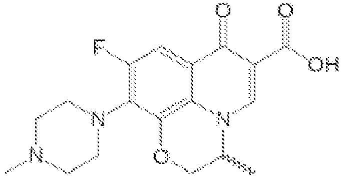

As noted above, levofloxacin is the levo isomer of ofloxacin.

Pefloxacin is a synthetic broad-spectrum fluoroquinolone antibacterial agent active against most gram-positive and gram-positive bacteria. Its chemical name is 1-ethyl-6-fluoro-7-(4-methylpiperazin-1-yl)-4-oxoquinoline-3-carboxylic acid and has the following structure:

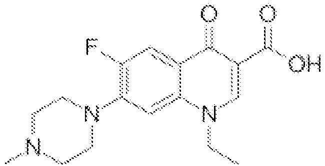

Rufloxacin is also known as 7-fluoro-6-(4-methylpiperazin-1-yl)-10-oxo-4-thia-1-azatricyclo[7.3.1.0^{5,13}]trideca-5(13),6,8,11-tetraene-11-carboxylic acid, and has the following chemical structure:

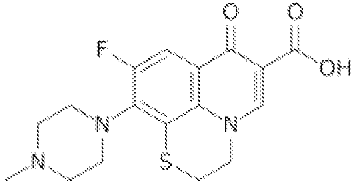

Balofloxacin is sold under the brand name Q-Roxin in Korea. It has the chemical name 1-cyclopropyl-6-fluoro-8-methoxy-7-(3-methylamino)piperidin-1-yl)-4-oxo-1,4-dihydroquinoline-3-carboxylic acid and the following chemical structure:

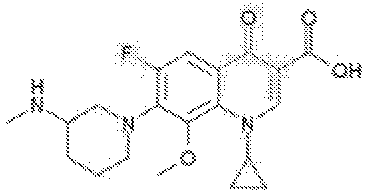

Grepafloxacin is an oral broad-spectrum fluoroquinolone also known as 1-cyclopropyl-6-fluoro-5-methyl-7-(3-methylpiperazin-1-yl)-4-oxoquinoline-3-carboxylic acid. It has the following chemical structure:

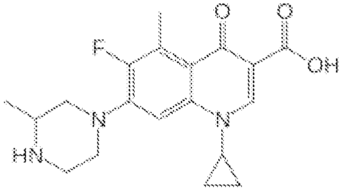

Pazufloxacin is sold in Japan under the brand names Pasil and Pazucross. It has the chemical name (2S)-6-(1-aminocyclopropyl)-7-fluoro-2-methyl-10-oxo-4-oxa-1-azatricyclo[7.3.1.0^{5,13}]trideca-5(13),6,8,11-tetraene-11-carboxylic acid and the following chemical structure:

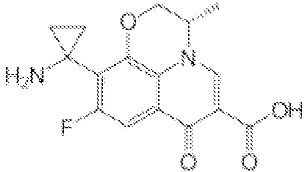

Sparfloxacin is indicated for treating community-acquired lower respiratory tract infections, and is available commercially under the names Sparcin and Zagam, among others. Its chemical name is 5-amino-1-cyclopropyl-7-[(3R,5S)-3,5-dimethylpiperazin-1-yl]-6,8-difluoro-4-oxoquinoline-3-carboxylic acid, and it has the following structure:

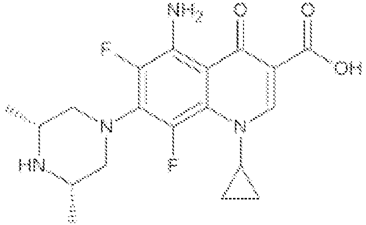

Sitafloxacin is a fluoroquinolone antibiotic that shows promise in the treatment of Buruli ulcer. Sitafloxacin is currently marketed in Japan under the trade name Gracevit and has the chemical name 7-[(7S)-7-amino-5-azaspiro[2.4]heptan-5-yl]-8-chloro-6-fluoro-1-[(1R,2S)-2-fluorocyclopropyl]4-oxoquinoline-3-carboxylic acid. The chemical structure of sitafloxacin is:

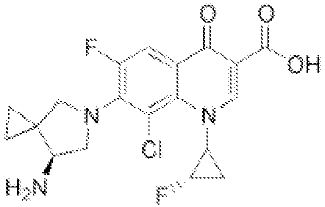

Besifloxacin is a fourth-generation fluoroquinolone antibiotic which is marketed as its hydrochloride salt under the trade name Besivance. It is indicated in the treatment of bacterial conjunctivitis caused by sensitive germs, as well as in the prevention of infectious complications in patients undergoing laser therapy for the treatment of cataracts. Besifloxacin has the chemical name of (R)-7-(3-aminohexahydro-1H-azepin-1-yl)-8-chloro-1-cyclopropyl-6-fluoro-1,4-dihydro-4-oxo-3-quinoline carboxylic acid and the following chemical structure:

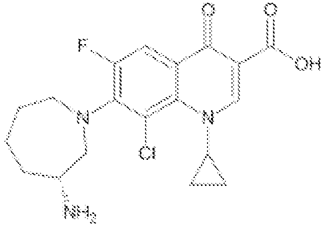

Delafloxacin is known under the trade name Baxdela, and is a fluoroquinolone antibiotic used to treat acute bacterial skin and skin structure infections. The injectable form of delafloxacin is sold as the meglumine salt of the active ingredient. The chemical name is 1,(6-amino-3,5-difluoropyridin-2-yl)-8-chloro-6-fluoro-7-(3-hydroxyazetidin-1-yl)-4-oxoquinoline-3-carboxylic acid, and it has the following structure:

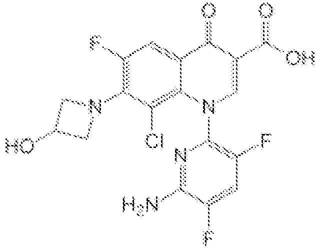

Ulifloxacin is also known as 6-fluoro-1-methyl-4-oxo-7-piperazin-1-yl-1H-[1,3]thiazeto[3,2-a]quinoline-3-carboxylic acid.

In various embodiments of the invention, the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.

In preferred embodiments, the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof. In more preferred embodiments, the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof. In most preferred embodiments, the fluoroquinolone antibiotic is selected from the group consisting of ciprofloxacin, levofloxacin, moxifloxacin, and pharmaceutically acceptable derivatives and prodrugs thereof.

Compounds for use according to the invention may be administered as the raw material but are preferably provided in the form of pharmaceutical compositions. The compounds may be used either as separate formulations or as a single combined formulation. When combined in the same formulation it will be appreciated that the two compounds must be stable and compatible with each other and the other components of the formulation.

Formulations of the invention include those suitable for oral, parenteral (including subcutaneous e.g. by injection or by depot tablet, intrathecal, intramuscular e.g. by depot and intravenous), and rectal or in a form suitable for administration by inhalation or insufflation administration. The most suitable route of administration may depend upon the condition and disorder of the patient. Preferably, the compositions of the invention are formulated for oral administration.

The formulations may conveniently be presented in unit dosage form and may be prepared by any of the methods well known in the art of pharmacy e.g. as described in *"*Remington: The Science and Practice of Pharmacy", Lippincott Williams and Wilkins, 21st Edition, (2005). Suitable methods include the step of bringing into association to active ingredients with a carrier which constitutes one or more excipients. In general, formulations are prepared by uniformly and intimately bringing into association the active ingredients with liquid carriers or finely divided solid carriers or both and then, if necessary, shaping the product into the desired formulation. It will be appreciated that when the two active ingredients are administered independently, each may be administered by a different means.

When formulated with excipients, the active ingredients may be present in a concentration from 0.1 to 99.5% (such as from 0.5 to 95%) by weight of the total mixture; conveniently from 30 to 95% for tablets and capsules and 0.01 to 50% (such as from 3 to 50%) for liquid preparations.

Formulations suitable for oral administration may be presented as discrete units such as capsules, cachets or tablets (e.g. chewable tablets in particular for paediatric administration), each containing a predetermined amount of active ingredient; as powder or granules; as a solution or suspension in an aqueous liquid or non-aqueous liquid; or as an oil-in-water liquid emulsion or water-in-oil liquid emulsion. The active ingredients may also be presented a bolus, electuary or paste.

A tablet may be made by compression or molding, optionally with one or more excipients. Compressed tablets may be prepared by compressing in a suitable machine the active ingredient in a free-flowing form such as a powder or granules, optionally mixed with other conventional excipients such as binding agents (e.g. syrup, acacia, gelatin, sorbitol, tragacanth, mucilage of starch, polyvinylpyrrolidone and/or hydroxymethyl cellulose), fillers (e.g. lactose, sugar, microcrystalline cellulose, maize-starch, calcium phosphate and/or sorbitol), lubricants (e.g. magnesium stearate, stearic acid, talc, polyethylene glycol and/or silica), disintegrants (e.g. potato starch, croscarmellose sodium and/or sodium starch glycolate) and wetting agents (e.g. sodium lauryl sulphate). Molded tablets may be made by molding in a suitable machine a mixture of the powdered active ingredient with an inert liquid diluent. The tablets may be optionally coated or scored and may be formulated so as to provide controlled release (e.g. delayed, sustained, or pulsed release, or a combination of immediate release and controlled release) of the active ingredients.

Alternatively, the active ingredients may be incorporated into oral liquid preparations such as aqueous or oily suspensions, solutions, emulsions, syrups or elixirs. Formulations containing the active ingredients may also be presented as a dry product for constitution with water or another suitable vehicle before use.

Such liquid preparations may contain conventional additives such as suspending agents (e.g. sorbitol syrup, methyl cellulose, glucose/sugar syrup, gelatin, hydroxymethyl cellulose, carboxymethyl cellulose, aluminium stearate gel and/or hydrogenated edible fats), emulsifying agents (e.g. lecithin, sorbitan mono-oleate and/or acacia), non-aqueous vehicles (e.g. edible oils, such as almond oil, fractionated coconut oil, oily esters, propylene glycol and/or ethyl alcohol), and preservatives (e.g. methyl or propyl p-hydroxybenzoates and/or sorbic acid).

Combinations for use according to the invention may be presented in a pack or dispenser device which may contain one or more unit dosage forms containing the active ingredients. The pack may, e.g. comprise metal or plastic foil, such as a blister pack. Where the compositions are intended for administration as two separate compositions these may be presented in the form of a twin pack.

Pharmaceutical compositions may also be prescribed to the patient in "patient packs" containing the whole course of treatment in a single package, usually a blister pack. Patient packs have an advantage over traditional prescriptions, where a pharmacist divides a patients' supply of a pharmaceutical from a bulk supply, in that the patient always has access to the package insert contained in the patient pack, normally missing in traditional prescriptions. The inclusion of the package insert has been shown to improve patient compliance with the physician's instructions.

The administration of the combination of the invention by means of a single patient pack, or patients packs of each composition, including a package insert directing the patient to the correct use of the invention is a desirable feature of this invention.

According to a further embodiment of the present invention there is provided a patient pack comprising at least one active of the combination according to the invention and an information insert containing directions on the use of the combination of the invention. In another embodiment of the invention, there is provided a double pack comprising in association for separate administration, an antimicrobial agent, preferably having biological activity against clinically latent microorganisms, and one or more of the compounds disclosed herein preferably having biological activity against clinically latent microorganisms.

The amount of active ingredients required for use in treatment will vary with the nature of the condition being treated and the age and condition of the patient, and will ultimately be at the discretion of the attendant physician. In general however, doses employed for adult human treatment will typically be in the range of 0.02 to 5000 mg per day, preferably 1 to 1500 mg per day. The desired dose may conveniently be presented in a single dose or as divided doses administered at appropriate intervals, e.g. as two, three or more sub-doses per day.

Suitable dosages and formulations for the administration of zidovudine are described in the product label for Retrovir^{®} oral solution or capsules which can be found at http://www.medicines.org.uk/emc/medicine/12444/SPC/Retrovir+250mg+Capsules/.

Suitable dosages and formulations for the administration of the fluoroquinolone antibiotic are described in the product labels for e.g. ciprofloxacin tablets or solution for infusion, levofloxacin tablets or solution for infusion, moxifloxacin tablets or solution for infusion, or ofloxacin tablets or infusion solution. Such labels can be readily found for the skilled person, including by searching for the fluoroquinolone antibiotic at https://www.medicines.org.uk/emc/browse-ingredients#

This information would therefore be readily obtained and understood by the person skilled in the art.

### Biological Tests

Test procedures that may be employed to determine the biological (e.g. bactericidal or antimicrobial) activity of the active ingredients include those known to persons skilled in the art for determining:
(a) bactericidal activity against clinically latent bacteria; and
(b) antimicrobial activity against log phase bacteria.

In relation to (a) above, methods for determining activity against clinically latent bacteria include a determination, under conditions known to those skilled in the art (such as those described in Nature Reviews, Drug Discovery 1, 895-910 (2002**)**, the disclosures of which are hereby incorporated by reference), of Minimum Stationary-cidal Concentration ("MSC") or Minimum Dormicidal Concentration ("MDC") for a test compound.

By way of example, WO2000028074 describes a suitable method of screening compounds to determine their ability to kill clinically latent microorganisms. A typical method may include the following steps:
(1) growing a bacterial culture to stationary phase;
(2) treating the stationery phase culture with one or more antimicrobial agents at a concentration and or time sufficient to kill growing bacteria, thereby selecting a phenotypically resistant sub-population;
(3) incubating a sample of the phenotypically resistant subpopulation with one or more test compounds or agents; and
(4) assessing any antimicrobial effects against the phenotypically resistant subpopulation.

According to this method, the phenotypically resistant sub-population may be seen as representative of clinically latent bacteria which remain metabolically active *in vivo* and which can result in relapse or onset of disease.

In relation to (b) above, methods for determining activity against log phase bacteria include a determination, under standard conditions (i.e. conditions known to those skilled in the art, such as those described in WO 2005014585, the disclosures of which document are hereby incorporated by reference), of Minimum Inhibitory Concentration ("MIC") or Minimum Bactericidal Concentration ("MBC") for a test compound. Specific examples of such methods are described below.

### Examples

### Example 1: In vitro synergistic effect of zidovudine (AZT) and ciprofloxacin

The chequerboard method used in Example 1 followed the protocols detailed in Antimicrob Chemo (2013) 68, 374-384. Zidovudine and ciprofloxacin were obtained from commercially available sources. The bacteria used were BAA2469 NDM-1 *Escherichia coli,* BAA2470 NDM-1 *Klebsiella pneumoniae,* BAA2471 NDM-1 *Escherichia coli,* BAA2472 NDM-1 *Klebsiella pneumoniae,* and NCTC13443 NDM-1 *Klebsiella pneumoniae.* All strains were obtained from a commercial source and log phase growth of the bacteria was carried out using methods known in the art.

The effects of the combination of the present invention were examined by calculating the fractional inhibitory concentration index (FICI) of each combination, as follows:
(MIC of drug A, tested in combination)/(MIC of drug A, tested alone)+(MIC of drug B, tested in combination)/(MIC of drug B, tested alone).

The interaction of the combination was defined as showing synergy if the FICI was ≤0.5, no interaction if the FICI was >0.5 but <4.0 and antagonism if the FICI was >4.0.

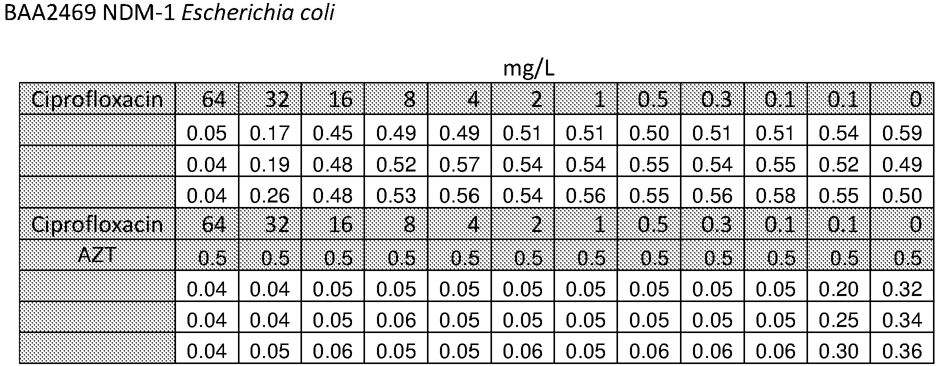

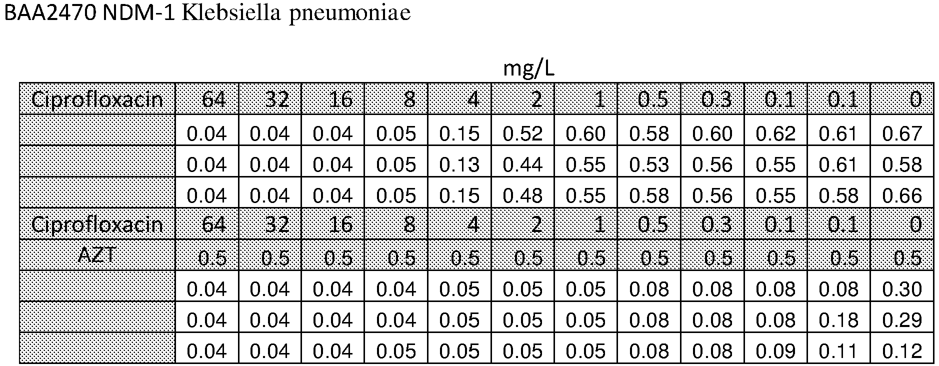

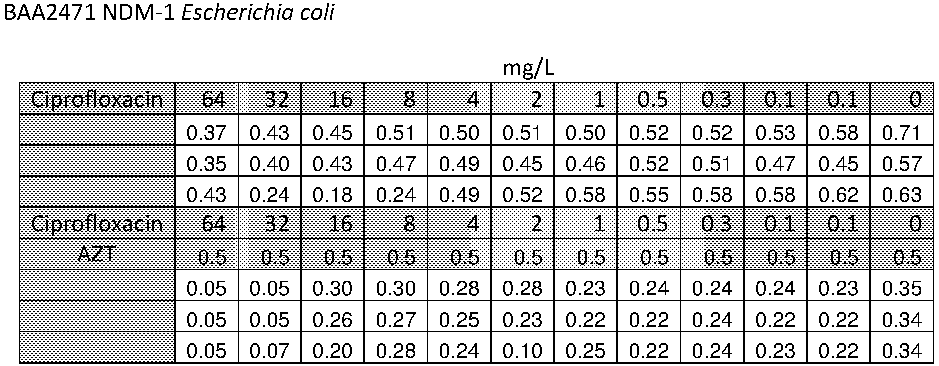

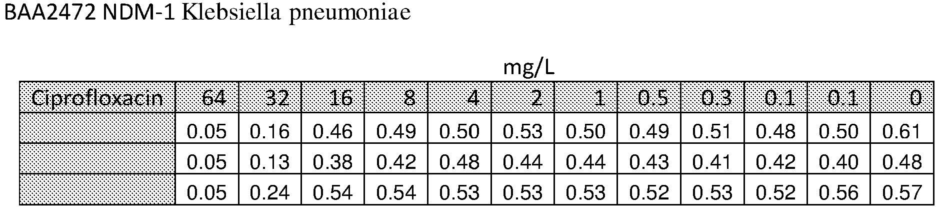

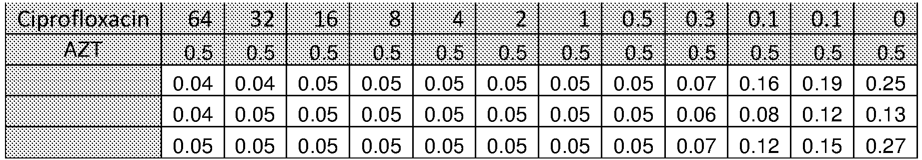

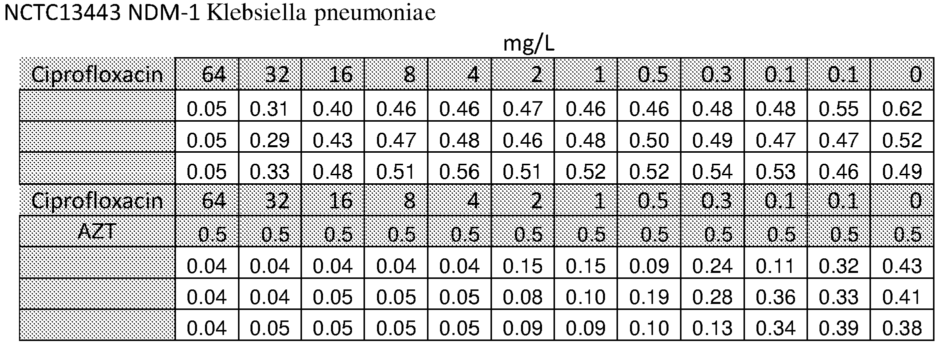

**Table 1. The reduction of ciprofloxacin MIC in combination with AZT**

| | MIC | | |
|---|---|---|---|
| | Ciprofloxacin | Ciprofloxacin + AZT | MIC fold reduction |
| BAA2469 | 64 | 0.125 | 512 |
| BAA2470 | 8 | 1 | 8 |
| BAA2471 | >64 | 32 | >4 |
| BAA2472 | 64 | 0.25 | 256 |
| NCTC13443 | 64 | 4 | 16 |

A MIC reduction of ≥4 fold indicates a synergistic effect. For ciprofloxacin + AZT, synergistic effects were observed for all of the strains tested, for example for strain BAA2469 NDM-1, ciprofloxacin MIC against the strain was 64 mg/L, but in combination with 0.5 mg/L AZT, its MIC reduced to 0.125 showing a 512-fold reduction.

### Example 2: In vitro synergistic effect of zidovudine in combination with levofloxacin

The method and bacterial strains were identical to Example 1. Zidovudine and levofloxacin were obtained from commercially available sources. The effects of the combination of the present invention were examined by calculating the MIC for each drug alone and in combination in the same manner as Example 1.

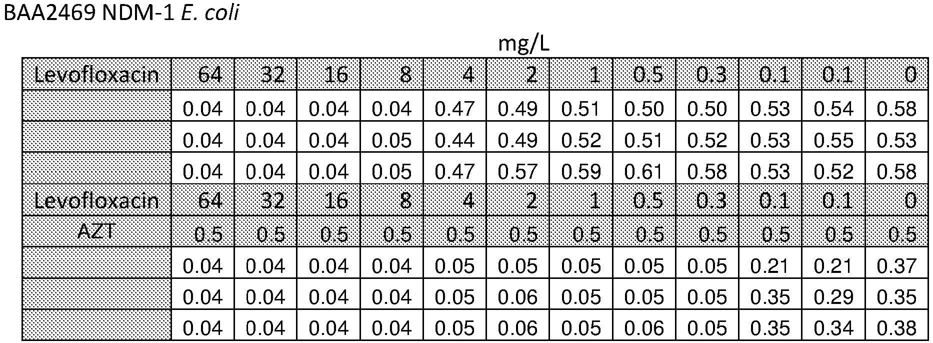

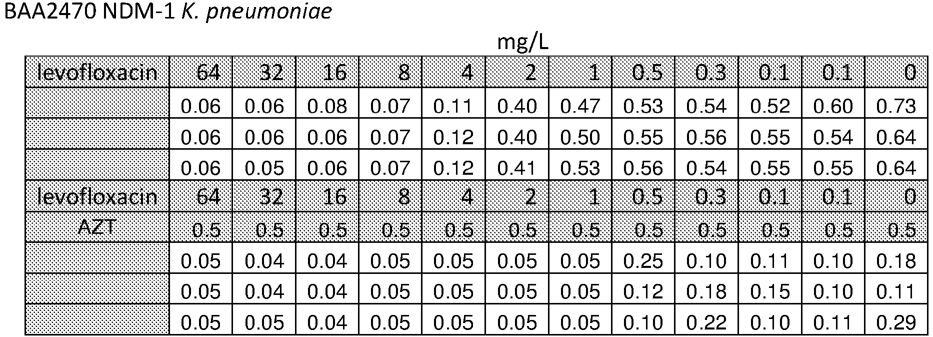

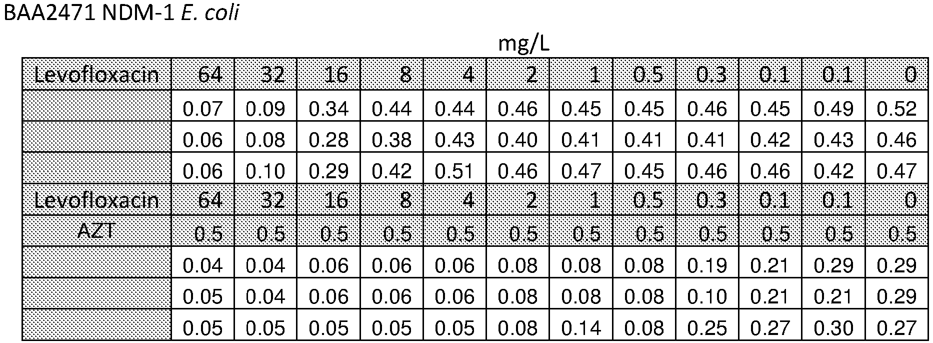

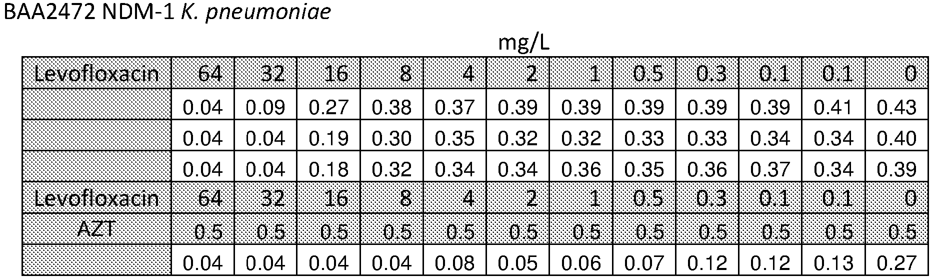

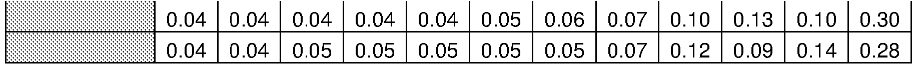

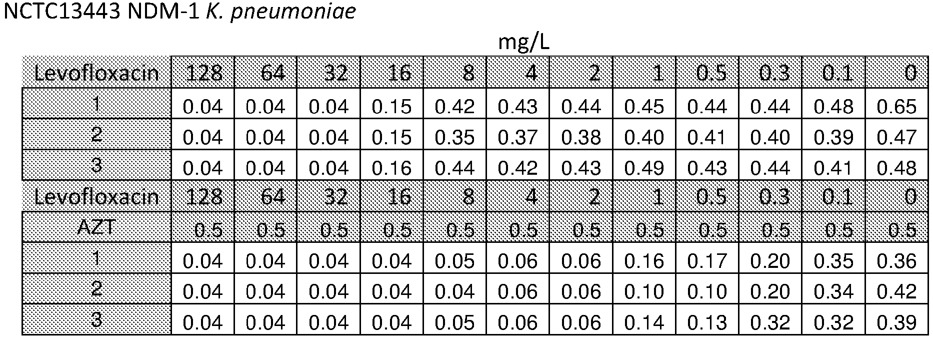

**Table 2. The reduction of levofloxacin MIC in combination with AZT**

| MIC | | | |
|---|---|---|---|
| | Levofloxacin | Levofloxacin + AZT | MIC fold reduction |
| BAA2469 | 8 | 0.25 | 32 |
| BAA2470 | 8 | 1 | 8 |
| BAA2471 | 32 | 4 | 8 |
| BAA2472 | 32 | 1 | 32 |
| NCTC13443 | 32 | 2 | 16 |

For levofloxacin + AZT, synergistic effects were observed for all of the strains tested, for example for strain BAA2469 NDM-1, levofloxacin MIC against the strain was high at 8 mg/L, in combination with 0.5 mg/L AZT, its MIC reduced to 0.25 showing a 32-fold reduction.

### Example 3: In vitro synergistic effect of zidovudine in combination with moxifloxacin

The method and bacterial strains were identical to Example 1. Zidovudine and moxifloxacin were obtained from commercially available sources. The effects of the combination of the present invention were examined by calculating the MIC for each drug alone and in combination in the same manner as Example 1.

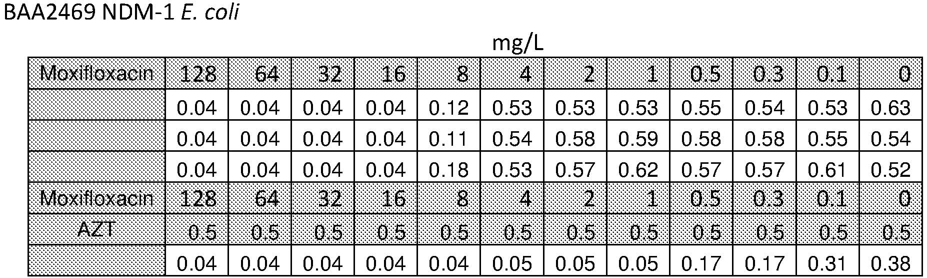

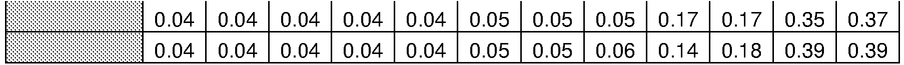

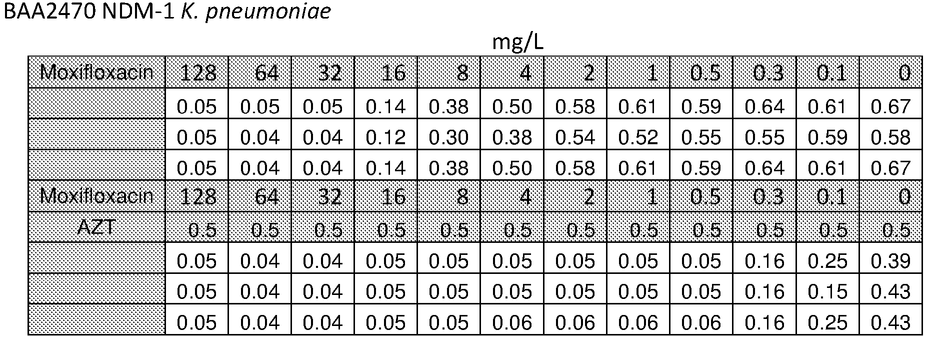

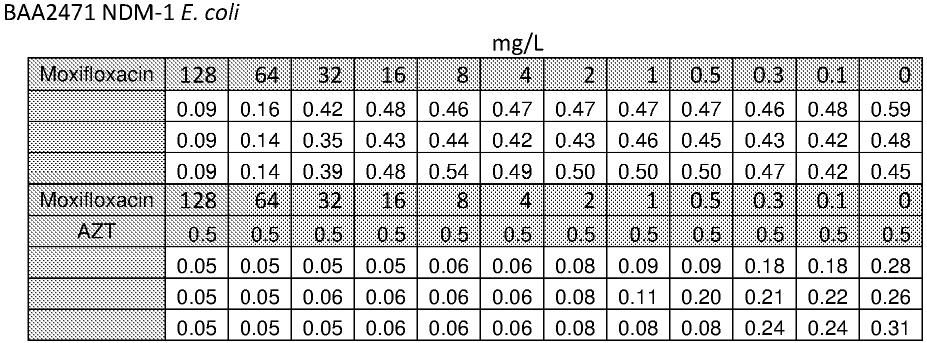

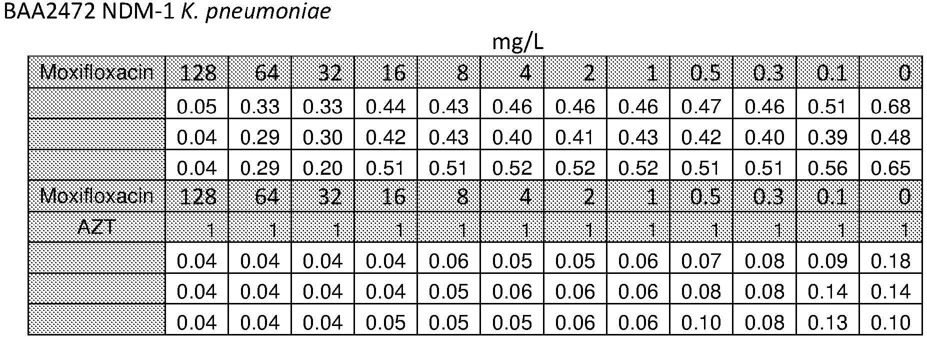

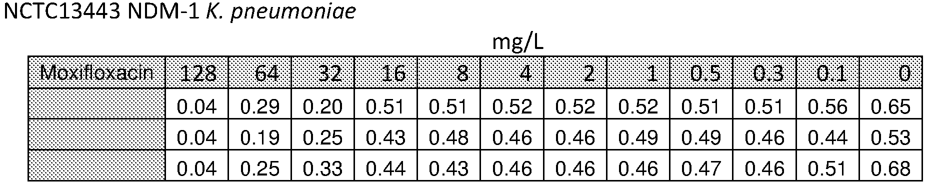

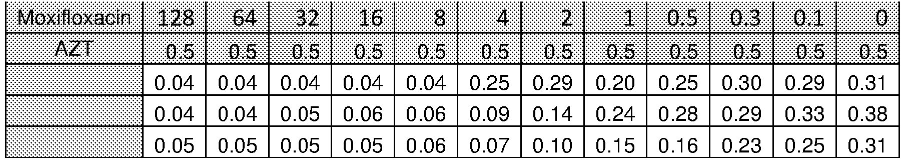

**Table 3. The reduction of moxifloxacin MIC in combination with AZT**

| | MIC | | |
|---|---|---|---|
| | Moxifloxacin | Moxifloxacin + AZT | MIC fold reduction |
| BAA2469 | 16 | 1 | 16 |
| BAA2470 | 32 | 0.5 | 64 |
| BAA2471 | 128 | 4 | 32 |
| BAA2472 | 128 | 1 | 128 |
| NCTC13443 | 128 | 8 | 16 |

For moxifloxacin + AZT, synergistic effects were seen against all of the strains tested, showing MIC reduction of moxifloxacin from 16 to 128-fold.

### Example 4: Further studies on zidovudine (AZT) in combination with levofloxacin

Further studies were conducted on AZT-levofloxacin combinations against carbapenem-resistant and ESBL-Phenotype *E.coli* and *K.pneumoniae* to determine the ability of the combinations to exhibit synergism. Synergism testing was completed against a defined set of levofloxacin-resistant *E. coli* and *K. pneumoniae* isolates according to methods described by the Clinical Microbiology Procedures Handbook (Leber AL, editor. 2016. Clinical Microbiology Procedures Handbook, 4th Ed. ASM Press, Washington, D. C.) using MIC testing reference methods published by the Clinical and Laboratory Standards Institute (CLSI. 2018. M07Ed11. Methods for dilution antimicrobial susceptibility tests for bacteria that grow aerobically; approved standard: eleventh edition. Clinical and Laboratory Standards Institute, Wayne, PA.; CLSI. 2019. M100Ed29. Performance standards for antimicrobial susceptibility testing: 29th informational supplement. Clinical and Laboratory Standards Institute, Wayne, PA.).

An isolate was categorized as carbapenem-resistant (CRE) if it was resistant to at least one of the following antimicrobials: doripenem, imipenem, or meropenem. An isolate was categorized as exhibiting an ESBL phenotype if the MIC value of at least 1 of the following antimicrobials was greater than 2 mg/L: aztreonam, ceftazidime, or ceftriaxone. A levofloxacin-resistant *Enterobacteriaceae* isolate was defined as any isolate displaying a levofloxacin MIC value of ≥2 mg/L.

The present application and invention further includes the subject
matter of the following numbered clauses:
1. A combination comprising zidovudine or a pharmaceutically acceptable derivative thereof and a fluoroquinolone antibiotic or a pharmaceutically acceptable derivative or prodrug thereof, for use in treating a microbial infection.
2. The combination for use according to clause 1 wherein the combination is for use in killing multiplying microorganisms associated with a microbial infection.
3. The combination for use according to clause 1 or clause 2, wherein the microbial infection is a bacterial infection and the microorganisms are bacteria.
4. The combination for use according to clause 3, wherein the infection is a gram negative bacterial infection.
5. The combination for use according to any one of clauses 1 to 4, wherein the infection is a urinary tract infection.
6. The combination for use according to any one of clauses 1 to 5, wherein the bacterial infection is caused by Enterobacteriaceae, preferably wherein the bacterial infection is caused by E.coli or Klebsiella pneumoniae.
7. The combination for use according to any one of clauses 1 to 6, wherein the infection is caused by a drug-resistant strain of bacteria.
8. The combination for use according to any one of clauses 1 to 7, wherein the use is in a subject other than an HIV-infected subject.
9. The combination for use according to any one of clauses 1 to 8, wherein the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.
10. The combination for use according to clause 9, wherein the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof.
11 . The combination for use according to clause 10, wherein the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof.
12. The combination for use according to clause 11 , wherein the fluoroquinolone antibiotic is selected from the group consisting of ciprofloxacin, levofloxacin, moxifloxacin, and pharmaceutically acceptable derivatives and prodrugs thereof.
13. A pharmaceutical composition comprising zidovudine or a pharmaceutically acceptable derivative thereof in combination with a fluoroquinolone antibiotic or a pharmaceutically acceptable derivative or prodrug thereof, and a pharmaceutically acceptable adjuvant, diluent or carrier, for use in the treatment of a microbial infection.
14. The pharmaceutical composition according to clause 13, wherein the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.
15. The pharmaceutical composition according to clause 14, wherein the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof.
16. The pharmaceutical composition according to clause 15, wherein the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof.
17. The pharmaceutical composition according to clause 16, wherein the fluoroquinolone antibiotic is selected from the group consisting of ciprofloxacin, levofloxacin, moxifloxacin, and pharmaceutically acceptable derivatives and prodrugs thereof.
18. A product comprising zidovudine or a pharmaceutically acceptable derivative thereof and a fluoroquinolone antibiotic compound or a pharmaceutically acceptable derivative or prodrug thereof, as a combined preparation for simultaneous, separate or sequential use in treating a microbial infection.
19. The product according to clause 18, wherein the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin and pharmaceutically acceptable derivatives and prodrugs thereof.
20. The product according to clause 19, wherein the fluoroquinolone antibiotic is selected from the group consisting of ciprofloxacin, gatifloxacin, gemifloxacin, levofloxacin, moxifloxacin, lomefloxacin, ofloxacin, pefloxacin, balofloxacin, grepafloxacin, and pharmaceutically acceptable derivatives and prodrugs thereof.
21 . The product according to clause 20, wherein the fluoroquinolone antibiotic is selected from the group consisting of ciprofloxacin, gatifloxacin, levofloxacin, moxifloxacin, ofloxacin, balofloxacin, grepafloxacin, and pharmaceutically acceptable derivatives and prodrugs thereof.
22. The product according to clause 21 , wherein the fluoroquinolone antibiotic is selected from the group consisting of ciprofloxacin, levofloxacin, moxifloxacin, and pharmaceutically acceptable derivatives and prodrugs thereof.

## Claims

1. A combination comprising zidovudine, or a pharmaceutically acceptable salt or solvate thereof, and a fluoroquinolone antibiotic, or a pharmaceutically acceptable salt or solvate thereof, for use in treating a bacterial infection caused by a drug-resistant strain of bacteria.

2. Zidovudine, or a pharmaceutically acceptable salt or solvate thereof, for use in treating a bacterial infection caused by a drug-resistant strain of bacteria in combination with a fluoroquinolone antibiotic, or a pharmaceutically acceptable salt or solvate thereof.

3. A fluoroquinolone antibiotic, or a pharmaceutically acceptable salt or solvate thereof, for use in treating a bacterial infection caused by a drug-resistant strain of bacteria in combination with zidovudine, or a pharmaceutically acceptable salt or solvate thereof.

4. The combination for use, zidovudine for use, or fluoroquinolone antibiotic for use according to any preceding claim, wherein the infection is a gram-negative bacterial infection.

5. The combination for use, zidovudine for use, or fluoroquinolone antibiotic for use according to any preceding claim, wherein the infection is a urinary tract infection.

6. The combination for use, zidovudine for use, or fluoroquinolone antibiotic for use according to any preceding claim, wherein the bacterial infection is caused by *Enterobacteriaceae,* optionally wherein the bacterial infection is caused by *E. coli* or *K*. *pneumoniae.*

7. The combination for use, zidovudine for use, or fluoroquinolone antibiotic for use according to claim 6, wherein the bacterial infection is caused by *K. pneumoniae.*

8. The combination for use, zidovudine for use, or fluoroquinolone antibiotic for use according to any preceding claim, wherein the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin, and pharmaceutically acceptable salts and solvates thereof.

9. The combination for use, zidovudine for use, or fluoroquinolone antibiotic for use according to any preceding claim, wherein the combination, zidovudine, or fluoroquinolone antibiotic is for use in treating a subject that is not a HIV-infected subject who is receiving zidovudine as part of their management of HIV.

10. A pharmaceutical composition comprising zidovudine, or a pharmaceutically acceptable salt or solvate thereof, a fluoroquinolone antibiotic, or a pharmaceutically acceptable salt or solvate thereof, and a pharmaceutically acceptable adjuvant, diluent or carrier, for use in the treatment of a bacterial infection caused by a drug-resistant strain of bacteria.

11. The pharmaceutical composition for use according to claim 10, wherein the composition is in the form of a tablet suitable for oral administration, or wherein the composition is suitable for parenteral administration.

12. A product comprising zidovudine, or a pharmaceutically acceptable salt or solvate thereof, and a fluoroquinolone antibiotic, or a pharmaceutically acceptable salt or solvate thereof, for use in the treatment of a bacterial infection caused by a drug-resistant strain of bacteria.

13. The product according to claim 12, wherein the product is a pack containing one or more unit dosage forms containing the active ingredients, optionally wherein the pack comprises dosage forms containing the active ingredients in separate formulations or in a single combined formulation.

14. The product according to claim 12 or claim 13, wherein the dosage forms are suitable for oral or parenteral administration.

15. The pharmaceutical composition for use according to claim 10 or claim 11, or the product according to any one of claims 12 to 14, wherein:
a) the infection is a urinary tract infection;
b) the bacterial infection is caused by *Enterobacteriaceae,* optionally wherein the bacterial infection is caused by *E. coli* or *K. pneumoniae,* further optionally wherein the bacterial infection is caused by *K. pneumoniae;*
c) the fluoroquinolone antibiotic is selected from the group consisting of ciprofloxacin, gatifloxacin, gemifloxacin, levofloxacin, moxifloxacin, lomefloxacin, norfloxacin, ofloxacin, pefloxacin, rufloxacin, balofloxacin, grepafloxacin, pazufloxacin, sparfloxacin, sitafloxacin, besifloxacin, delafloxacin, ulifloxacin, and pharmaceutically acceptable salts and solvates thereof; and/or
d) the use is a use in treating a subject that is not a HIV-infected subject who is receiving zidovudine as part of their management of HIV.
